# EUROPEAN PATENT APPLICATION

(11) **EP 3 701 943 A1**
(43) Date of publication of application: **02.09.2020**
(21) Application number: 19159413.4
(22) Date of filing: 26.02.2019
(51) Int. Cl.: A61K 9/14, A61K 9/16, A61K 31/282, A61K 31/337, A61K 31/704, A61P 35/00

(54) **DELAYED DELIVERY OF ANTICANCER DRUGS**

(71) Applicant: CAPNOMED GmbH, 78658 Zimmern (DE)
(72) Inventor: Sahoo, Ranjita, 47574 Goch (DE)
(74) Representative: Eisenführ Speiser

(57) **Abstract**

The present invention relates to an anti-cancer delivery system with a delayed release of one or more chemotherapeutic agent(s), a mixture for use in the treatment of cancer comprising such a delivery system and a method for producing such a delivery system.

## Description

The present invention relates to an anti-cancer delivery system, a mixture for use in the treatment of cancer comprising such a delivery system and a method for producing such a delivery system.

Cancer is the second leading cause of death globally, and is responsible for an estimated 9.6 million deaths in 2018. Globally, about 1 in 6 deaths is due to cancer. Among the most common cancers are lung cancer (2.09 million cases, 1.76 million deaths in 2018), breast cancer (2.09 million cases, 627,000 deaths in 2018), colorectal cancer (1.80 million cases, 862,000 deaths in 2018), prostate cancer (1.28 million cases) and stomach cancer (1.03 million cases, 783,000 deaths in 2018).

Metastases are new pathological sites spread by a cancerous (e.g. malignant) tumour. Malignant tumours are capable to invade into adjacent and distant tissue, e.g. by invasion into the blood circulation, followed by invasion into another site, where another tumour (i.e. a metastasis) may grow. Likewise, such tumours may spread along various tissues, which complicates especially a surgical removal of the tumour.

The cancer site as used herein is the part or cavity of the body, i.e. the respective tissue or part of a tissue, where cancer cells located.

Particularly in view of cancer sites, the abdomen is an important region of the body. The abdomen stretches from the thorax at the thoracic diaphragm to the pelvis at the pelvic brim. The region occupied by the abdomen is termed the abdominal cavity and contains many organs such as the stomach, the small intestine and the colon, the liver, the gall bladder and the pancreas. The abdominal cavity is coated by an extensive membrane, called the peritoneum, which covers the abdominal wall and the pelvic walls (parietal peritoneum) as well as the included organs (visceral peritoneum).

A special form of metastases are abdominal, particularly peritoneal metastases. As the cancer cells are attached to the outside of several organs and tissues but reach into the area of the peritoneum (and are thus classified as abdominal or particularly peritoneal), these metastases are more difficult to reach with medication via the blood circulation. Cancer cells may therefore "escape" into the peritoneum and/or peritoneal cavity. Still, in many cases, these cancer sites are covered by the peritoneum, i.e. are positioned between the respective organ or tissue and the peritoneum.

The terms "peritoneal metastasis" and "peritoneal cancer" as used herein may be understood as synonyms. Both terms comprise cancer cells of a primary or a secondary site of the host's body.

The treatment options for cancer strongly depend on several factors such as the involved organs, the cause of cancer and further complicating factors as well as the age and health status of the patient.

Although many limitations, for various cancer types, a systemic chemotherapy is applied as a standard of care. However, several tissues have a poor vascularisation and can thus be poorly reached by a systemically applied chemotherapy. The therapy thus results in a poor performance. As a counteractive measure, higher doses and/or higher volumes of the chemotherapeutic agent(s) could be applied transport higher amounts of the chemotherapeutic agent into the poorly vascularised tissues. However, increasing concentrations also increase the risk of dangerous adverse effects, renal toxicity, neurotoxicity or cardiac toxicity.

As an improvement to systemic chemotherapy, local chemotherapy can be applied to patients with tumour sites in poorly vascularized tissues. The delivery of the chemotherapeutically active substance is thereby localised to a desired tissue or region.

Such treatment options have recently emerged particularly for cancer sites in the poorly vascularized peritoneal cavity:
Possible treatment options for peritoneal metastasis are e.g. Hyperthermic Intraperitoneal Chemotherapy (HIPEC) or Pressurized Intraperitoneal Aerosol Chemotherapy (PIPAC). In some cases, electro-precipitation may also be an option.

Hyperthermic Intraperitoneal Chemotherapy (HIPEC) is a treatment usually performed after surgery when all visible cancer sites in the abdomen are surgically removed. A liquid heated chemotherapy is applied to the peritoneal cavity, bathing all reachable organs and their surfaces. With this treatment, any remaining cancer cells shall be killed. However, this treatment is a time-consuming process with higher mortality rates.

Pressurized Intraperitoneal Aerosol Chemotherapy (PIPAC) is a palliative treatment of peritoneal metastasis. PIPAC is a drug delivery method applying chemotherapeutic drugs, in form of an aerosol, under pressure into the abdominal cavity. The aerosol is usually applied within the closed abdominal cavity, i.e. during a laparoscopy. PIPAC is a significantly time saving therapy compared to other available techniques like HIPEC.

In many cases, however, sole chemotherapy is not sufficient for treating cancer and a surgery, in which the cancer cells are removed, becomes necessary or even the only remaining option. Surgeries generally provide risks to patients such as (internal) bleedings, infections and stress for the cardiovascular system during anaesthesia. Nevertheless, surgeries are usually necessary if performed. However, to prevent the risks involved, unnecessary surgeries should be avoided.

A drawback of the surgical removal of cancer cells is that in several cases, some cancer cells or cancer cell clusters remain in the body, as they were too small to be seen or detected during surgery. These cancer cells or cancer cells clusters usually tend to be very aggressive, i.e. are rapidly growing and likely to spread in form of metastases.

Furthermore, healing includes the rapid growth of tissue in proximity of the wound, which is usually triggered or accompanied by a mixture of endogenous growth stimulants. Such stimulants may also promote growth of the remaining cancer cells or cancer cell clusters.

As the body is weakened by performing the surgery, a systemic chemotherapy to kill the remaining cancer cells or cancer cell clusters is not an option. Also, local chemotherapy often cannot be performed, as the substances themselves usually interfere with the healing process or as the local application of the chemotherapeutic agent is performed under pressure (e.g. in case of PIPAC) or other (physical) interactions with the tissue affected by surgery.

Moreover, even if the chemotherapeutic agents reach their destination, their concentration must be low enough to ensure proper wound healing but at the same time high enough to fight the remaining cancer cells or cancer cell clusters. Thus, their concentration needs to be tightly regulated overtime (concentrations may and should be increased with increasing healing progress) and be analysed to intervene if it becomes necessary.

Therefore, it was the primary object of the present invention to provide new and improved treatment measures for treating cancer or in connection with treating cancer, particularly shortly after a surgery for removing a tumour, preferably to provide new and improved delivery systems to be used in the treatment of cancer, wherein one or more, particularly preferably all, of the above-mentioned drawbacks can be avoided or overcome.

The primary object of the present invention is achieved by an anti-cancer agent delivery system, comprising or consisting of
a) one, two, three or more chemotherapeutic agents
   optionally dissolved in a solvent, preferably the same solvent, and
b) a matrix surrounding component a), wherein the matrix is suitable to be degraded in the human body, preferably in the peritoneal cavity, and
   wherein the matrix comprises
   i) one or more surfactants,
   ii) one or more lipids and
   iii) one or more fillers,
   wherein in an in vitro assay, comprising or consisting of the following steps:
   1) providing the anti-cancer agent delivery system, wherein the amount of the chemotherapeutic agent(s) is pre-determined,
   2) adding the anti-cancer agent delivery system to a pre-determined amount of liquid medium which does not contain the chemotherapeutic agent(s) of the delivery system, and
   3) analysing the amount of the chemotherapeutic agent(s) released from the delivery system into the medium after a pre-determined time or after pre-determined times,
the matrix prevents the release of more than 5 wt.-%, 4 wt.-%, 3 wt.-%, 2 wt.-%, 1 wt.-% of component a), based on the total weight of component a), to the medium for at least 7 days, preferably at least 10 days, particularly preferably at least 15 days, at least 20 days, preferably at least 30, at least 40, at least 50, at least 60, at least 70, at least 80 or at least 90 days after the addition of the anti-cancer agent delivery system in step 2),
for use in the treatment of cancer in a subject, wherein the treatment comprises the application of the delivery system to the human subject by administration of the delivery system by direct introduction into the abdomen. Before introduction, the delivery system can be diluted.

Preferably in the in vitro assay, the total delivery system is enveloped with a membrane, which allows the medium and the chemotherapeutic agent(s), however, not the delivery system as such to pass. The term "enveloped" as used herein describes a condition in which the delivery system is completely surrounded by the membrane. The membrane may be any means suitable to allow the medium and the chemotherapeutic agent(s), however, not the delivery system as such to pass. The membrane may be of any suitable material. Preferably, the membrane is a polyvinylidene fluoride (PVDF) membrane, a polyester (PE) membrane, a polyether sulfone (PES) membrane, a nylon membrane or a polycarbonate membrane. Preferably, the pore size of the membrane is in the range of from 0.01 µm to 0.1 µm.

Preferably, the enveloped delivery system is then added to the liquid medium.

Preferably, the liquid medium is water, saline, phosphate buffer saline (PBS), tris buffered saline (TBS), 4-(2-hydroxyethyl)-1-piperazineethanesulfonic acid (HEPES) buffer, or a mixture consisting of 50 vol.% of an artificial peritoneal dialysis fluid and 50 vol.% of saline and thus does not contain the chemotherapeutic agent(s) of the delivery system. Typically, an isotonic saline solution containing 9 g of sodium chloride per liter is used as saline. An artificial peritoneal dialysis fluid may have the following composition:

| **Composition of the final solution after mixing in mmol/L** | |
|---|---|
| Anhydrous glucose (C₆H₁₂O₆) | 126 |
| Sodium (Na⁺) | 132 |
| Magnesium (Mg⁺⁺) | 1.25 |
| Calcium (Ca⁺⁺) | 0.25 |
| Chloride (Cl⁻) | 95 |
| Bicarbonate (HCO₃⁻) | 25 |
| Lactate | 15 |
| Osmolarity | 395 mOsmol/L |

Preferably in the in vitro assay, the medium is continuously stirred at 50 to 80 rpm.

Typically, samples of e.g. 2 ml of the medium are taken 5 days, 7 days, 10 days, 15 days, 20 days, 30 days, 40 days, 50 days, 60 days, 70 days, 80 days and/or 90 days after the addition of the anti-cancer agent delivery system and preferably, the collected medium is replaced by the same amount (e.g. 2 ml) of the same medium (which was freshly prepared).

Subsequently, the collected samples are analysed for their content of the chemotherapeutic agent(s) by means of HPLC, however other suitable techniques are also known to a skilled person and may also be used.

As the amount of the chemotherapeutic agent(s) is pre-determined, as is the amount of the liquid medium, the amount of the chemotherapeutic agent(s) measured in the sample taken (the volume of which is known) can be translated to the amount of the chemotherapeutic agent(s) in the liquid medium and thus to the released amount (e.g. in wt.-%) of the chemotherapeutic agent(s) from the delivery system.

It is further preferred that in the in vitro assay,
the matrix prevents the release of more than 5 wt.-% of the chemotherapeutic agent(s) for at least 7 days, preferably at least 10 days, particularly preferably at least 15 days
and
an increase of the amount of released chemotherapeutic agent(s) can be measured until 30 days, preferably 40 days, 50 days, 60 days, 70 days, 80 days, 90 days after the addition of the anti-cancer agent delivery system.

The term "an increase of the amount of released chemotherapeutic agent(s) can be measured until 30 days" is meant to be understood such that the delivery system releases the chemotherapeutic agent(s) at least until 30 days after the addition of the anti-cancer agent delivery system to the liquid medium. The release may also be interrupted once or several times until the end of 30 days, however, has to be resumed once or several times until the end of 30 days. The same applies for other time points, e.g. 40 days, 50 days, 60 days, 70 days, 80 days or 90 days, accordingly.

Preferably the subject is a human, preferably a human suffering from cancer, such as a cancer of any body cavity, preferably a cancer type selected from the group consisting of gastrointestinal cancer, gynaecological cancer, prostate cancer, peritoneal metastasis, leukemia, lymphoma, soft-tissue sarcoma, pseudomyxoma peritonei, yolk sack tumor, peritoneal cancer, multiple myeloma, breast cancer, bladder cancer, lung cancer, thyroid cancer, Kaposi's sarcoma, particularly preferably ovarian cancer, peritoneal metastasis colorectal cancer or stomach cancer and other cancers.

The treatment as described herein can comprise or consist of a parenteral administration of the delivery system, preferably an intraperitoneal administration of the delivery system.

The treatment as described herein preferably comprises an administration of the delivery system before, during or after surgery, preferably by systemic application, preferably an oral or intravenous application, or by local application, preferably an intraperitoneal application.

Preferably, the one, two, three or more, preferably dissolved, chemotherapeutic agents are surrounded by the matrix by means of a core-shell principle, i.e. the preferably dissolved chemotherapeutic agent(s) form(s) one core, which is surrounded by the matrix.

Alternatively, and also preferably, the, preferably dissolved, chemotherapeutic agent(s) may also form several compartments defined and separated by each other by the matrix. In this case, it is preferred that all the compartments are completely surrounded by the matrix.

The treatment of cancer in a human subject can be any kind of treatment of cancer. Particularly, the treatment is directed to cancer in a body cavity. Preferably, the treatment is directed to a cancer type selected from the group consisting of gastrointestinal cancer, gynaecological cancer, prostate cancer, peritoneal metastasis, leukemia, lymphoma, soft-tissue sarcoma, pseudomyxoma peritonei, yolk sack tumor, peritoneal cancer, multiple myeloma, breast cancer, bladder cancer, lung cancer, thyroid cancer, Kaposi's sarcoma, particularly preferably ovarian cancer, peritoneal metastasis colorectal cancer or stomach cancer and other cancers.

The treatment as described herein can be directed to reducing tumour size and the amount of tumour sites. Tumour sites includes the primary tumour as well as metastases and further tumour reproductions. The treatment can also be directed to prevent or delay cancer recurrence after tumour removal or partial tumour removal, preferably after a surgical tumour removal.

The anti-cancer agent delivery system may be used in several treatments, as e.g. PIPAC as described above.

It is of particular advantage, that due to the delayed release, the anti-cancer agents in the anti-cancer agent delivery system do not (significantly) impair wound healing.

It is preferred that the treatment comprises one, two, three or more administration(s) of the delivery system, particularly preferably one administration of the delivery system.

Preferably, the, one, two, several or all chemotherapeutic agents used within the scope of the present invention is/are selected from the group consisting of chemotherapeutic agents used against a cancer type selected from the group consisting gastrointestinal cancer, gynaecological cancer, prostate cancer, peritoneal metastasis, leukemia, lymphoma, soft-tissue sarcoma, pseudomyxoma peritonei, yolk sack tumor, peritoneal cancer, multiple myeloma, breast cancer, bladder cancer, lung cancer, thyroid cancer, Kaposi's sarcoma, particularly preferably ovarian cancer, peritoneal metastasis colorectal cancer or stomach cancer and other cancers.

Typically, and preferably such chemotherapeutic agents are selected from the group consisting of cisplatin, doxorubicin, paclitaxel and oxaliplatin and a combination thereof.

Preferably, the chemotherapeutic agent(s) is/are dissolved in a solvent. In some cases, the solvent can also be added in a later step during a process of manufacture of the delivery system or the solvent may be partially or completely removed during such a process, as it may be the case for some solid forms of the delivery system.

The solvent(s) for dissolving the chemotherapeutic agent(s) is/are preferably solvents that are pharmaceutically acceptable. Preferably, the solvents include polyethylene glycol, such as polyethylene glycol 400; acetonitrile; N-methylpyrrolidone; dimethyl sulfoxide; ethanol; dimethylacetamide; propylene glycol; DMSO and combinations thereof. According to the present invention, the chemotherapeutic agent(s) may be solved in one or more solvents, i.e. in a single solvent or a solvent mixture. Furthermore, in case of more than one agent, the agents can either be solved in the same solvent / solvent mixture or can be solved in different solvents / solvent mixtures.

In addition to the chemotherapeutic agent(s), component a) can further include one or more immunomodulatory agent(s), preferably such as curcumin, lutein, flavonoids like hesperidin, apigenin, hesperetin, aioene, arctigenin, β-carotene, Lycopene epigallocatechin-3-gallate, ginsan, glabridin and guinic acid. Furthermore, component a) may further comprise one or more antibodies, one or more viruses, one or more vitamin(s), one or more salt(s), one or more ions and/or one or more further adjuvants and inhibitors.

What is said with regard to the release of chemotherapeutic agent(s) herein also applies for the other compounds which may be present in component a). Where amounts, e.g. of release or of present substance, are indicated for the chemotherapeutic agent(s), these amounts may also apply for the other compounds separately, i.e. the amounts indicated for the chemotherapeutic agent(s) do not include the further compounds.

It is preferred that component a) comprises one chemotherapeutic agent but no further agent.

It is also preferred that component a) comprises two or more chemotherapeutic agents, but no further agent.

Moreover, it is preferred that component a) comprises one chemotherapeutic agent and one immunomodulatory agent.

It is also preferred that component a) comprises one chemotherapeutic agent and two or more immunomodulatory agents.

Furthermore, it is preferred that component a) comprises two or more chemotherapeutic agents and one immunomodulatory agent.

Further, it is also preferred that component a) comprises two or more chemotherapeutic agents and two or more immunomodulatory agents.

Preferably, the amount of the or, respectively, each chemotherapeutic agent is contained in a range of from 0.01 to 70 wt.-%, preferably 0.1 to 50 wt.-%, based on the total weight of the delivery system.

Additionally or alternatively, the amount of the or, respectively, each chemotherapeutic agent is preferably contained in a range of from 0.01 mg to 2000 mg, preferably of from 0.1 mg to 1500 mg, particularly preferably of from 1 mg to 1000 mg, preferably this indication refers to the total amount administered during one application, preferably as a powder or suspended in physiological solutions like saline.

Additionally or alternatively, during one application, an amount of 100 to 5000 mg, preferably 150 to 4000 mg, particularly preferably 200 to 3000 mg, especially preferably 250 to 2500 mg of the delivery system is administered. The delivery system may be diluted in saline or any other physiological solution, which is pharmaceutically acceptable, before administration.

Preferably, the or one, two, three or all administration(s) of the delivery system is/are performed with an assisting tool selected from the group consisting of microneedles, spray devices, angio-injectors, or any combination thereof, preferably with a nebulizer, spraying gun or spray catheter, particularly preferably the assisting tool is a laparoscopic nebulizer, especially preferably the laparoscopic nebulizer known as Capnopen, preferably as described in US 9,511,197 B2.

Preferably, the, one or all surfactants i) of the matrix is/are selected from cationic surfactants, non-ionic surfactants and/or anionic surfactants, preferably the, one or all surfactants i) of the matrix is/are selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid.

Additionally, or alternatively, the, one or all lipids ii) of the matrix is/are preferably selected from the group consisting of triglycerides, diglycerides, monoglycerides, phospholipids, lipoproteins and fatty acids. Particularly preferably, the, one or all lipids ii) of the matrix is/are selected from the group consisting of Glyceryl Monostearate, Glycerol Mono-oleate, Medium-Chain Triglycerides, Sorbitan MonoStearate, Sorbitan MonoPalmitate, Sorbitan MonoLaurate, Sorbitan Monooleate, Sorbitan Trioleate, Polyoxyethylene 20 Sorbitan mono stearate, Polyoxyethylene 20 Sorbitan mono palmitate, Polyoxyethylene 20 Sorbitan Mono Laurate, Polyoxyethylene 20 Sorbitan Mono Oleate, Propylene glycol monocaprylate, Propylene glycol caprylate, Propylene glycol Monolaurate, Oleoyl macrogolglycerides, Linoleoyl macrogolglycerides, Polyoxyl 35 castor oil, Polyoxyl 40 hydrogenated castor oil, Polyoxyl 60 hydrogenated castor oil, Capryl alcohol, Capri alcohol, Lauryl alcohol, Myristyl alcohol, Palmityl alcoholl, Stearyl alcohol, Arachidiyl alcohol, Behenyl alcohol, Oleyl alcohol, Cetostearyl alcohol, α-Linolenic acid, Myristoleic acid, Palmitoleic acid, Oleic acid, Linoleic acid.

Additionally or alternatively the, one or all fillers iii) of the matrix can be selected from the group consisting of lactose, mannitol, lactulose, glucose, trehalose, xylose, sorbitol, sucrose and/or consisting of polymers such as carbopol polymers; polyethylenglycols, such as PEG 4000, preferably with a molecular weight of 600 g/mol or more; polyvinyl alcohols; polyvinylpyrrolidone; hydrocolloids, preferably carrageenan, chitosan or algenic acid; hydrogels; cellulose and its derivatives such as microcrystalline cellulose, cross-linked cellulose, non-ionic cellulose, hydroxypropyl cellulose, hydroxypropylmethyl cellulose, methyl cellulose, carboxymethyl cellulose, cellulose ethers such as ethyl cellulose or hydroxyethyl cellulose, anionic ether derivatives such as sodium carboxymethyl cellulose; starch, preferably fully gelatised starch, partially gelitised starch, native starch, cross-linked starch or pre-gelatised starch.

The delivery system according to the invention can for example be present in form of a powder (e.g. micron, submicron or nanosized), pellets, granules, an aerosol, i.e. as liquid or solid particle(s) in a gas or mixture of gases, a suspension or an emulsion. If present in liquid or in solid form, such as powder, pellets or granules, the delivery system can be mixed with a gas or a mixture of gases to produce an aerosol. Alternatively, if present in solid form, the delivery system can be dissolved or dispersed in a suitable solvent / solvent or diluent fluid or in suitable physiological fluid mixture before application to the subject in need thereof.

The present invention also relates to a mixture for use in the treatment of cancer, such as any cancer of a body cavity. Preferably the cancer type is selected from the group consisting of gastrointestinal cancer, gynaecological cancer, prostate cancer, peritoneal metastasis, leukemia, lymphoma, soft-tissue sarcoma, pseudomyxoma peritonei, yolk sack tumor, peritoneal cancer, multiple myeloma, breast cancer, bladder cancer, lung cancer, thyroid cancer, Kaposi's sarcoma, particularly preferably ovarian cancer, peritoneal metastasis colorectal cancer or stomach cancer and other cancers.

The mixture according to the invention can for example be present in form of a powder, pellets, granules, an aerosol, i.e. as liquid or solid particle(s) in a gas or mixture of gases, a suspension or an emulsion. Preferably the mixture is present in a form which can be administered to a human subject. Further preferably, a pharmaceutically acceptable solvent can be added to the mixture to ease the application to the subject, preferably human subject.

Preferably, the mixture is a mixture for use as described for the delivery system according to the invention.

It is also preferred that the mixture comprises a delivery system according to the invention. Further preferably, the mixture comprises two, three, four, five or more different delivery systems according to the invention. The delivery systems can for example differ in the respective chemotherapeutic agent / the mixture of chemotherapeutic agents and/or in the composition of the respective matrix.

Furthermore, the present invention relates to a method for producing a delivery system as defined herein comprising or consisting of the steps
a) providing the one, two, three or more chemotherapeutic agents,
   a1) optionally: dissolving the chemotherapeutic agent(s) in a solvent, preferably the same solvent,
b) providing the components of the matrix, wherein the matrix preferably consists of or comprises
   i) one or more surfactants,
   ii) one or more lipids and
   iii) one or more fillers,
   preferably wherein the, one or all surfactants i) of the matrix is/are selected from cationic surfactants, non-ionic surfactants and/or anionic surfactants, preferably the, one or all surfactants i) of the matrix is/are selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid,
   and/or
   preferably wherein the, one or all lipids ii) of the matrix is/are selected from the group consisting of triglycerides, diglycerides, monoglycerides, phospholipids, lipoproteins and fatty acids,
   particularly preferably, the, one or all lipids ii) of the matrix is/are selected from the group consisting of Glyceryl Monostearate, Glycerol Mono-oleate, Medium-Chain Triglycerides, Sorbitan MonoStearate, Sorbitan MonoPalmitate, Sorbitan MonoLaurate, Sorbitan Monooleate, Sorbitan Trioleate, Polyoxyethylene 20 Sorbitan mono stearate, Polyoxyethylene 20 Sorbitan mono palmitate, Polyoxyethylene 20 Sorbitan Mono Laurate, Polyoxyethylene 20 Sorbitan Mono Oleate, Propylene glycol monocaprylate, Propylene glycol caprylate, Propylene glycol Monolaurate, Oleoyl macrogolglycerides, Linoleoyl macrogolglycerides, Polyoxyl 35 castor oil, Polyoxyl 40 hydrogenated castor oil, Polyoxyl 60 hydrogenated castor oil, Capryl alcohol, Capri alcohol, Lauryl alcohol, Myristyl alcohol, Palmityl alcoholl, Stearyl alcohol, Arachidiyl alcohol, Behenyl alcohol, Oleyl alcohol, Cetostearyl alcohol, α-Linolenic acid, Myristoleic acid, Palmitoleic acid, Oleic acid, Linoleic acid,
   and/or
   preferably wherein the, one or all fillers iii) of the matrix is/are selected from the group consisting of lactose, mannitol, lactulose, glucose, trehalose, xylose, sorbitol, sucrose and/or consisting of polymers such as carbopol polymers;
   polyethylenglycols, such as PEG 4000, preferably with a molecular weight of 600 g/mol or more; polyvinyl alcohols; polyvinylpyrrolidone; hydrocolloids, preferably carrageenan, chitosan or algenic acid; hydrogels; cellulose and its derivatives such as microcrystalline cellulose, cross-linked cellulose, non-ionic cellulose, hydroxypropyl cellulose, hydroxypropylmethyl cellulose, methyl cellulose, carboxymethyl cellulose, cellulose ethers such as ethyl cellulose or hydroxyethyl cellulose, anionic ether derivatives such as sodium carboxymethyl cellulose; starch, preferably fully gelatised starch, partially gelitised starch, native starch, cross-linked starch or pre-gelatised starch,
c) mixing the provided components i), ii) and iii),
d) optionally: processing the mixture obtained in step c) by spray drying, fluid bed drying, granulation, spheronization or melt casting.

The mixing step c) is preferably performed by low shear mixing, preferably at 500 to 2,000 rpm. Additionally, or alternatively, the processing step, if present, can be performed at a temperature in a range of from 20 to 100 °C, preferably 25 to 85 °C, particularly preferably 50 to 80 °C.

Also described herein is a method for detecting the release of an anti-cancer agent from an anti-cancer agent delivery system, wherein the anti-cancer agent delivery system comprises or consists of
a) one, two, three or more chemotherapeutic agents,
   optionally dissolved in a solvent or combination of solvents, preferably the same solvent, and
b) a matrix surrounding component a), wherein the matrix is suitable to be degraded in the human body, preferably in the peritoneal cavity,
preferably, wherein the anti-cancer agent delivery system is a delivery system according to the invention
wherein the method comprises or consists of the following steps:
i) administering an anti-cancer agent delivery system to a subject in need of anti-cancer treatment before, during or after a surgery, preferably a surgical removal of a tumour, more preferably during a surgical removal of a tumour,
   preferably wherein the anti-cancer delivery system is administered intraperitoneally,
ii) measuring the concentration of the anti-cancer agent(s) after the administration in step i), preferably not later than 1 day, not later than 12 hours, not later than 6 hours, not later than 4 hours, not later than 3 hours, or not later than 2 hours after the administration in step i),
   preferably in a first sample taken from the subject, particularly preferably in a first blood sample taken from the subject,
iii) comparing the measured concentration with a pre-determined threshold for the concentration of the anti-cancer agent(s),
iv) measuring the concentration of the anti-cancer agent(s) after a pre-determined time after the administration in step i),
   preferably in a second sample taken from the subject, particularly preferably in a second blood sample taken from the subject,
   wherein the measurement is performed after the measurement in step ii),
v) comparing the measured concentration of step iv) with the pre-determined threshold for the concentration of the anti-cancer agent(s) and with the value obtained in step ii),
vi) optionally: repeating the measurement and the comparison, preferably for one, two, three, four, five or more times after further pre-determined times after the administration in step i) in one, two, three, four, five or more further samples taken from the subject,
   wherein such a measurement is performed after the measurement in step iv),
vii) determining the success of the anti-cancer agent delivery system, wherein a system is successful if all of the following conditions are met:
   a) the comparison in step iii) reveals that the concentration measured in step ii) is at or below the pre-determined threshold,
   b) the comparison in step v) or, where applicable, at least one of the comparisons in step v) and the comparisons as repeated in step vi) reveals that the concentration measured in step iv) or, where applicable, in a measurement as repeated in step vi) is higher than the pre-determined threshold,
      and
   c) the comparison in step v) or, where applicable, at least one of the comparison in step v) and the comparisons as repeated in step vi) reveals that the concentration measured in step iv) or, where applicable, in a measurement as repeated in step vi) is higher than the concentration measured in step ii).

Preferably, the pre-determined threshold is a value as it can be obtained from a subject without having received an anti-cancer agent which is in the anti-cancer agent delivery system administered in step i).

It is further preferred in the described method that the pre-determined time in step iv) is at least 7 days, preferably at least 10 days, particularly preferably at least 15 days, and/or preferably, the further pre-determined time in step vi) is at least 20 days, preferably at least 30, at least 40, at least 50, at least 60, at least 70, at least 80 or at least 90 days.

The described method may further comprise the steps of measuring the concentration of the anti-cancer agent(s) and preferably of comparing the measured concentration with a pre-determined threshold for the concentration of the anti-cancer agent(s), wherein this step is performed before the anti-cancer agent delivery system is administered.

The method for detecting the release according to the invention is preferably not applied to a patient *per se* but applied on the previously taken samples. Thus, the method according to the invention is preferably a method for detecting the release of an anti-cancer agent from an anti-cancer agent delivery system in a first sample as well as in a second sample taken from a subject, as described herein. Particularly preferably, the samples are blood, blood plasma, blood serum, peritoneal fluid or tissue samples, especially blood or peritoneal fluid samples, taken from the subject. Further, a combination of two types of samples is possible in clinical practice, e.g. blood and tissue samples or blood and peritoneal fluid.

Also described herein is an anti-cancer agent delivery system, as described herein, *per se*, i.e. independent of its use. The anti-cancer agent delivery system comprises or consists of
a) one, two, three or more chemotherapeutic agents
   optionally dissolved in a solvent, preferably the same solvent, and
b) a matrix surrounding component a), wherein the matrix is suitable to be degraded in the human body, preferably in the peritoneal cavity.

The matrix preferably consists of or comprises
i) one or more surfactants,
ii) one or more lipids and
iii) one or more fillers,
preferably wherein the, one or all surfactants i) of the matrix is/are selected from the group consisting of cationic surfactants, non-ionic surfactants and/or anionic surfactants, preferably the, one or all surfactants i) of the matrix is/are selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid,
and/or
preferably wherein the, one or all lipids ii) of the matrix is/are selected from the group consisting of triglycerides, diglycerides, monoglycerides, phospholipids, lipoproteins and fatty acids,
particularly preferably, the, one or all lipids ii) of the matrix is/are selected from the group consisting of Glyceryl Monostearate, Glycerol Mono-oleate, Medium-Chain Triglycerides, Sorbitan MonoStearate, Sorbitan MonoPalmitate, Sorbitan MonoLaurate, Sorbitan Monooleate, Sorbitan Trioleate, Polyoxyethylene 20 Sorbitan mono stearate, Polyoxyethylene 20 Sorbitan mono palmitate, Polyoxyethylene 20 Sorbitan Mono Laurate, Polyoxyethylene 20 Sorbitan Mono Oleate, Propylene glycol monocaprylate, Propylene glycol caprylate, Propylene glycol Monolaurate, Oleoyl macrogolglycerides, Linoleoyl macrogolglycerides, Polyoxyl 35 castor oil, Polyoxyl 40 hydrogenated castor oil, Polyoxyl 60 hydrogenated castor oil, Capryl alcohol, Capri alcohol, Lauryl alcohol, Myristyl alcohol, Palmityl alcoholl, Stearyl alcohol, Arachidiyl alcohol, Behenyl alcohol, Oleyl alcohol, Cetostearyl alcohol, α-Linolenic acid, Myristoleic acid, Palmitoleic acid, Oleic acid, Linoleic acid,
and/or
preferably wherein the, one or all fillers iii) of the matrix is/are selected from the group consisting of lactose, mannitol, lactulose, glucose, trehalose, xylose, sorbitol, sucrose and/or consisting of polymers such as carbopol polymers; polyethylenglycols, such as PEG 4000, preferably with a molecular weight of 600 g/mol or more; polyvinyl alcohols; polyvinylpyrrolidone; hydrocolloids, preferably carrageenan, chitosan or algenic acid; hydrogels; cellulose and its derivatives such as microcrystalline cellulose, cross-linked cellulose, non-ionic cellulose, hydroxypropyl cellulose, hydroxypropylmethyl cellulose, methyl cellulose, carboxymethyl cellulose, cellulose ethers such as ethyl cellulose or hydroxyethyl cellulose, anionic ether derivatives such as sodium carboxymethyl cellulose; starch, preferably fully gelatised starch, partially gelitised starch, native starch, cross-linked starch or pre-gelatised starch.

The system as described herein preferably is a delivery system which would be considered successful according to step vii) of the method for detecting the release of an anti-cancer agent from an anti-cancer agent delivery system as described herein. I.e. when used in such a method according to the invention, all of the following conditions are met:
a) the comparison in step iii) reveals that the concentration measured in step ii) is at or below the pre-determined threshold,
b) the comparison in step v) or, where applicable, at least one of the comparisons in step v) and the comparisons as repeated in step vi) reveals that the concentration measured in step iv) or, where applicable, in a measurement as repeated in step vi) is higher than the pre-determined threshold,
   and
c) the comparison in step v) or, where applicable, at least one of the comparison in step v) and the comparisons as repeated in step vi) reveals that the concentration measured in step iv) or, where applicable, in a measurement as repeated in step vi) is higher than the concentration measured in step ii).

What is said herein with regard to the delivery system for use, also applies for preferred embodiments of the delivery system *per se.*

Preferred embodiments and further aspects of the present invention also emerge from the attached patent claims and the following examples, wherein the present invention is not limited to these examples.

### Examples:

### Example 1: Low shear mixing

| **Ingredient** | **Wt.-%** |
|---|---|
| Carboxymethyl cellulose | 5 |
| Glyecryl behenate | 30 |
| Lactose | 28 |
| PEG 4000 | 29 |
| Tween 80 | 5 |
| water | 2 |
| Cisplatin | 1 |
| Sum | 100 |

All the components are provided. 1000 mg of Cisplatin are dispersed in 29 g of PEG 4000 and 30 g of glyceryl behenate at 80 °C. This solution was used as a granulating fluid for the mixture of 28 g Lactose, 2 g water and 5 g carboxymethyl cellulose under stirring at 500 rpm for 25 min to obtain granules with an average diameter of 0.1 - 0.5 cm.

### Example 2: Fluid bed drying

| **Ingredient** | **Wt.-%** |
|---|---|
| Stearic acid | 20 |
| Lactose | 30 |
| Oxaliplatin | 1 |
| Mannitol | 46 |
| Poloxamer 188 | 2 |
| Hydroxypropylmethyl cellulose | 1 |
| Water at 90 degree | q.s. |
| Sum | 100 |

1 g of Oxaliplatin, 2 g of poloxamer, 20 g of stearic acid, 1 g of hydroxypropylmethyl cellulose and 30 g of lactose are mixed in a fluid bed dryer to obtain mixture A. 46 g of Mannitol were dissolved in warm water and was sprayed on the dried mixture A till uniform granules are obtained. The complete process was carried out at an inlet temperature of 120 °C and an outlet temperature of 70 °C.

### Example 3: Granulation

| **Ingredient** | **Wt.-%** |
|---|---|
| Ethyl cellulose | 47 |
| Povidone | 20 |
| Palmitic acid | 30 |
| Poloxamer 188 | 2 |
| Doxorubicin | 1 |
| Sum | 100 |

47 g of Ethyl cellulose, 20 g of povidone, 1 g of doxorubicin and 2 g of poloxamer are mixed. 30 g of Palmitic acid are melted at 50-80 °C and added to the mixture. All the components are subjected to hot melt extrusion at 90 °C to form pellets, which are then milled to a suitable smaller size.

### Example 4: Spheronization

| **Ingredient** | **Wt.-%** |
|---|---|
| Hydroxypropylmethyl cellulose | 15 |
| Poloxamer 188 | 5 |
| PEG 4000 | 45 |
| Lactose | 24 |
| Oxaliplatin | 1 |
| Water | 5 |
| Ethanol | 5 |
| Sum | 100 |

15 g of Hydroxypropylmethyl cellulose, 5 g of poloxamer 188, 24 g of lactose and 1 g of Oxaliplatin are mixed in form of a powder. 5 g of water and 5 g of ethanol were added to the powder mixture to moisten the powder bed. 45 g of melted PEG 4000 at 80 °C were added and admixed to mixture. The mixture was immediately extruded using a Caleva extruder.

The extrudate is subsequently shaped into round granules by spheronization which was performed at 1500 rpm and at room temperature.

88.5 g of granules are obtained.

### Example 5: Crystal mix 1

| **Ingredient** | **Wt.-%** |
|---|---|
| Povidone | 2 |
| Polysorbate 80 | 8 |
| Lactose | 20 |
| Fully gelatinised starch | 10 |
| Triglyceride | 20 |
| PEG 4000 | 34 |
| Paclitaxel | 1 |
| Water | 5 |
| Sum | 100 |

1000 mg of Paclitaxel are mixed into a powder bed of 2 g of povidone, 20 g of lactose and 10 g of fully gelatinised starch. 5 g of water at 50 °C and 8 g of polysorbate 80 were sprayed on the powder bed to moist the mass. In another beaker, 20g of triglycerides are melted and used as granulating fluid (number-1). Simultaneously, 34 g of PEG 4000 are melted and used as a granulating fluid (number-2). In this way, the particles are attached and covered to each other either by a lipid or by PEG or a mixture thereof. The complete granulation process is performed at 1500 rpm.

95.6 g of crystals are obtained.

### Example 6: Crystal mix 2

| **Ingredient** | | **Wt.-%** |
|---|---|---|
| Mixture 1 | triglyceride | 20 |
| | Paclitaxel | 1 |
| | Water | 49 |
| | lactose | 20 |
| | Tween 80 | 5 |
| | Polyvinyl alcohol | 5 |
| Mixture 2 | Glyceryl behenate | 20 |
| | Phospholipid | 2 |
| | Cisplatin | 1 |
| | Poloxamer | 1 |
| | Mannitol | 27 |
| | Water | 49 |

### Mixture 1:

1 g of Paclitaxel was dissolved in 20 g of triglycerides in one beaker and at 80 °C to obtain phase A. In another beaker, 5 g of tween 80 and 5 g of polyvinyl chloride were dissolved in 49 g of water and 20 g of lactose were dispersed therein at 80 °C to obtain phase B. Phase B is added to phase A under stirring at 1500-2000 rpm for 5 min and the solution is immediately cooled in an ice bath for 30 min and subsequently centrifuged to obtain a pellet.

### Mixture 2:

1 g of Cisplatin and 2 g phospholipids were dissolved in 20 g of glyceryl behenate in one beaker and at 80 °C to obtain phase A. In another beaker, 27 g of mannitol and 1 g poloxamer were dissolved in 49 g of water at 80 °C to obtain phase B. Phase B is added to phase A under stirring at 1500-2000 rpm for 5 min and the solution is immediately cooled in an ice bath for 30 min and subsequently centrifuged to obtain a pellet.

The pellets obtained from mixture A and mixture B were homogeneously mixed by vortexing and subsequently lyophilised to obtain a crystal mix. This method is used for preparing an anti-cancer agent delivery system of two different anti-cancer agents which can be administered simultaneously.

### Example 7: Melt casting

| **Ingredient** | **Wt.-%** |
|---|---|
| Hydroxypropylmethyl cellulose | 20 |
| Mannitol | 8 |
| Ethanol | 30 |
| Glycerol | 10 |
| Triglyceride | 5 |
| Poloxamer | 1 |
| Cisplatin | 1 |
| Water | 25 |
| Sum | 100 |

1 g of Cisplatin is dissolved in 25 g of water, and mixed with 1 g of poloxamer and 30 g of ethanol. Subsequently, 10 g of glycerol was added and the mixture was stirred at 1000 rpm for 10 min. To this solution, 20 g of HPMC were added and the mixture was warmed to 80 °C. Further, 5 g triglycerides were added. A film is casted on casting belt and was cut in suitable size.

94 g of the solidified product are then taken from the mould.

### Example 8: Lipid nanoparticles

| **Ingredient** | **Wt.-%** |
|---|---|
| Cetyl palmitate | 15 |
| Phospholipid | 1 |
| Oxaliplatin | 1 |
| Water | 76 |
| Poloxamer 188 | 1 |
| mannitol | 5 |
| Polyvinylpyrrolidone | 1 |
| Sum | 100 |

1 g of Oxaliplatin was dissolved in 15 g of cetyl palmitate and mixed with 1 g of phospholipid in a beaker and at 85 °C to obtain mixture A. In another beaker, 1 g of poloxamer 188, 1 g of polyvinylpyrrolidone and 5 g of mannitol were dissolved in 76 g of water at 80 °C to obtain mixture B. Phase B is added to phase A under stirring at 1500-2000 rpm for 5 min.

The warm emulsion mixture is then passed through a high-pressure homogeniser either at 3 cycles at 1000 bar or 3 cycles at 1200 bar or 3 cycles at 1500 bar, according to the desired particle size. Upon homogenisation, the warm emulsion is immediately cooled in an ice bath for 30 min.

### Application example 1: Release profile

Composition A was prepared according to Example 1.

Composition B was prepared according to Example 4.

Composition C was prepared according to Example 8.

Composition D was prepared according to Example 5.

The obtained delivery systems (compositions A-D) were enveloped with a PVDF membrane with a pore size of approx. 0.1 µm, which allows the medium and the chemotherapeutic agent(s), however, not the delivery system as such to pass. The enveloped delivery systems are each added to a separate medium mixture of 25 ml of artificial peritoneal dialysis fluid and 25 ml saline, which is warmed to 37 °C and continuously stirred at 80 rpm.

5 days, 7 days, 10 days, 30 days, 60 days and 90 days after the addition of the enveloped delivery systems, a sample of 2 ml of the medium is taken and replaced with fresh medium.

The anti-cancer agent content in the samples was analysed by applying HPLC.

For all compositions, no anti-cancer agent content could be measured in the medium until 5 days, thus, there was no release. After 7 days, a release could be measured for compositions B, C and D. For composition B, however, the release was lower than 5 wt.- %.

The peak release of composition A, B and C was between 30 and 60 days. The peak release of composition D was around day 10. A release until day 30 was measured for all compositions. A release until day 60 was measured for compositions A, B and C.

## Claims

1. Anti-cancer agent delivery system, comprising or consisting of
a) one, two, three or more chemotherapeutic agents
optionally dissolved in a solvent, preferably the same solvent, and
b) a matrix surrounding component a), wherein the matrix is suitable to be degraded in the human body, preferably in the peritoneal cavity, and wherein the matrix comprises
i) one or more surfactants,
ii) one or more lipids, and
iii) one or more fillers,
wherein in an in vitro assay, comprising or consisting of the following steps:
1) providing the anti-cancer agent delivery system, wherein the amount of the chemotherapeutic agent(s) is pre-determined,
2) adding the anti-cancer agent delivery system to a pre-determined amount of liquid medium, preferably a mixture consisting of 50 vol.% of an artificial peritoneal dialysis fluid and 50 vol.% of saline, and which does not contain the chemotherapeutic agent(s) of the delivery system, and
3) analysing the amount of the chemotherapeutic agent(s) released from the delivery system into the medium after a pre-determined time or after pre-determined times,
the matrix prevents the release of more than 5 wt.-% of the chemotherapeutic agent(s) for at least 7 days, preferably at least 10 days, particularly preferably at least 15 days, at least 20 days, preferably at least 30, at least 40, at least 50, at least 60, at least 70, at least 80 or at least 90 days after the addition of the anti-cancer agent delivery system in step 2),
for use in the treatment of cancer in a human subject, wherein the treatment comprises the application of the delivery system to the human subject by administration of the delivery system by direct introduction into the abdomen.

2. Delivery system as defined in claim 1 for use according to claim 1, wherein one, the, two, several or all chemotherapeutic agents is/are selected from the group consisting of chemotherapeutic agents used against a cancer type selected from the group consisting of gastrointestinal cancer, gynaecological cancer, prostate cancer, peritoneal metastasis, leukemia, lymphoma, soft-tissue sarcoma, pseudomyxoma peritonei, yolk sack tumor, peritoneal cancer, multiple myeloma, breast cancer, bladder cancer, lung cancer, thyroid cancer, Kaposi's sarcoma, particularly preferably ovarian cancer, peritoneal metastasis colorectal cancer or stomach cancer, preferably selected from the group consisting of cisplatin, doxorubicin, paclitaxel and oxaliplatin.

3. Delivery system as defined in claim 1 or 2 for use according to claim 1, wherein the, one or all surfactants i) of the matrix is/are selected from cationic surfactants, non-ionic surfactants and/or anionic surfactants, preferably the, one or all surfactants i) of the matrix is/are selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid.

4. Delivery system as defined in any one of claims 1 to 3 for use according to claim 1, wherein the, one or all lipids ii) of the matrix is/are selected from the group consisting of triglycerides, diglycerides, monoglycerides, phospholipids, lipoproteins and fatty acids.

5. Delivery system as defined in any one of claims 1 to 4 for use according to claim 1, wherein the, one or all fillers iii) of the matrix is/are selected from the group consisting of lactose, mannitol, lactulose, glucose, trehalose, xylose, sorbitol, sucrose and/or consisting of polymers such as carbopol polymers; polyethylenglycols, such as PEG 4000, preferably with a molecular weight of 600 g/mol or more; polyvinyl alcohols; polyvinylpyrrolidone; hydrocolloids, preferably carrageenan, chitosan or algenic acid; hydrogels; cellulose and its derivatives such as microcrystalline cellulose, cross-linked cellulose, non-ionic cellulose, hydroxypropyl cellulose, hydroxypropylmethyl cellulose, methyl cellulose, carboxymethyl cellulose, cellulose ethers such as ethyl cellulose or hydroxyethyl cellulose, anionic ether derivatives such as sodium carboxymethyl cellulose; starch, preferably fully gelatised starch, partially gelitised starch, native starch, cross-linked starch or pre-gelatised starch.

6. Delivery system as defined in any one of claims 1 to 5 for use according to claim 1, wherein in the in vitro assay, the total delivery system is enveloped with a membrane, when added to the liquid medium, which allows the medium and the chemotherapeutic agent(s), however, not the delivery system as such to pass,
wherein the enveloped delivery system is added to the medium, which is continuously stirred at 50 to 80 rpm,
wherein samples of 2 ml of the medium are taken 5 days, 7 days, 10 days, 15 days, 20 days, 30 days, 40 days, 50 days, 60 days, 70 days, 80 days and/or 90 days after the addition of the anti-cancer agent delivery system and the collected medium is replaced by the same amount of the same medium and
wherein the collected samples are analysed for their content of the chemotherapeutic agent(s) by means of HPLC.

7. Delivery system as defined in any one of claims 1 to 6 for use according to claim 1, wherein in the in vitro assay,
the matrix prevents the release of more than 5 wt.-% of the chemotherapeutic agent(s) for at least 7 days, preferably at least 10 days, particularly preferably at least 15 days
and
an increase of the amount of released chemotherapeutic agent(s) can be measured until 30 days, preferably 40 days, 50 days, 60 days, 70 days, 80 days, 90 days after the addition of the anti-cancer agent delivery system.

8. Delivery system as defined in any one of claims 1 to 7 for use according to claim 1, wherein the amount of the or, respectively, each chemotherapeutic agent is contained in a range of from 0.01 to 70 wt.-%, preferably 1 to 50 wt.-%, based on the total weight of the delivery system.

9. Delivery system as defined in any one of claims 1 to 8 for use according to claim 1, wherein the treatment is directed to a cancer type selected from the group consisting of gastrointestinal cancer, gynaecological cancer, prostate cancer, peritoneal metastasis, leukemia, lymphoma, soft-tissue sarcoma, pseudomyxoma peritonei, yolk sack tumor, peritoneal cancer, multiple myeloma, breast cancer, bladder cancer, lung cancer, thyroid cancer, Kaposi's sarcoma, particularly preferably ovarian cancer, peritoneal metastasis colorectal cancer or stomach cancer.

10. Delivery system as defined in any one of claims 1 to 8 for use according to claim 1 or 9, wherein the or one administration of the delivery system is performed with an assisting tool selected from the group consisting of microneedles, spray devices, angioinjectors, or any combination thereof, preferably with a nebulizer, spraying gun or spray catheter.

11. Mixture for use in the treatment of cancer, preferably a cancer type selected from the group consisting of gastrointestinal cancer, gynaecological cancer, prostate cancer, peritoneal metastasis, leukemia, lymphoma, soft-tissue sarcoma, pseudomyxoma peritonei, yolk sack tumor, peritoneal cancer, multiple myeloma, breast cancer, bladder cancer, lung cancer, thyroid cancer, Kaposi's sarcoma, particularly preferably ovarian cancer, peritoneal metastasis colorectal cancer or stomach cancer, comprising a delivery system as defined in any one of claims 1 to 8.

12. Mixture as defined in claim 11 for use according to claim 11, comprising two, three, four, five or more different delivery systems as defined in claims 1 to 8,
wherein the delivery systems differ in the respective chemotherapeutic agent / the mixture of chemotherapeutic agents and/or in the composition of the respective matrix.

13. Delivery system as defined in any one of claims 1 to 8 for use according to any one of claims 1, 9 or 10 or mixture as defined in claim 11 or 12 for use according to claim 11, wherein the treatment is directed to prevent or delay cancer recurrence after a surgical tumour removal.

14. Method for producing a delivery system as defined in any one of claims 1 to 8 comprising or consisting of the steps
a) providing the one, two, three or more chemotherapeutic agents,
a1) optionally: dissolving the chemotherapeutic agent(s) in a solvent, preferably the same solvent,
b) providing the components of the matrix, wherein the matrix consists of or comprises
i) one or more surfactants,
ii) one or more lipids, and
iii) one or more fillers,
preferably wherein the, one or all surfactants i) of the matrix is/are selected from cationic surfactants, non-ionic surfactants and/or anionic surfactants, preferably the, one or all surfactants i) of the matrix is/are selected from the group consisting of Macrogol glycerol ricinoleate, Polyoxyl-35-castor oil, Polysorbates, Poloxamers, Span, Macrogol 15 Hydroxystearate, Polyoxyl 15 Hydroxystearate, Polyethoxylated 12-hydroxystearic acid,
and/or
preferably wherein the, one or all lipids ii) of the matrix is/are selected from the group consisting of triglycerides, diglycerides, monoglycerides, phospholipids, lipoproteins and fatty acids,
and/or
preferably wherein the, one or all fillers iii) of the matrix is/are selected from the group consisting of lactose, mannitol, lactulose, glucose, trehalose, xylose, sorbitol, sucrose and/or consisting of polymers such as carbopol polymers; polyethylenglycols, such as PEG 4000, preferably with a molecular weight of 600 g/mol or more; polyvinyl alcohols; polyvinylpyrrolidone; hydrocolloids, preferably carrageenan, chitosan or algenic acid; hydrogels; cellulose and its derivatives such as microcrystalline cellulose, cross-linked cellulose, non-ionic cellulose, hydroxypropyl cellulose, hydroxypropylmethyl cellulose, methyl cellulose, carboxymethyl cellulose, cellulose ethers such as ethyl cellulose or hydroxyethyl cellulose, anionic ether derivatives such as sodium carboxymethyl cellulose; starch, preferably fully gelatised starch, partially gelitised starch, native starch, cross-linked starch or pre-gelatised starch,
c) mixing the provided components of steps a) and b),
d) optionally: processing the mixture obtained in step c) by spray drying, fluid bed drying, granulation, spheronization or melt casting.
